(19)

Europäisches
Patentamt

European
Patent Office

Office européen
des brevets

(11) **EP 3 217 391 A1**

(12) **EUROPEAN PATENT APPLICATION**

(43) Date of publication:
**13.09.2017 Bulletin 2017/37**

(51) Int Cl.:
***G10K 11/30*** *(2006.01)*

(21) Application number: **17159574.7**

(22) Date of filing: **07.03.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **09.03.2016 JP 2016045885**

(71) Applicant: **Seiko Epson Corporation Tokyo 160-8801 (JP)**

(72) Inventors:
• **Yoshida, Kazuki**
  **Nagano, 392-8502 (JP)**
• **Nakamura, Tomoaki**
  **Nagano, 392-8502 (JP)**
• **Kiyose, Kanechika**
  **Nagano, 392-8502 (JP)**

(74) Representative: **Hoffmann Eitle Patent- und Rechtsanwälte PartmbB Arabellastraße 30 81925 München (DE)**

(54) **ULTRASONIC DEVICE, ULTRASONIC MODULE, AND ULTRASONIC MEASUREMENT APPARATUS**

(57)    An ultrasonic device (22) includes an ultrasonic transceiver (46) having a flat, ultrasonic wave transmitting/receiving surface and an acoustic lens provided on the ultrasonic wave transmitting/receiving surface. The acoustic lens has a first acoustic lens layer on the side facing away from the ultrasonic wave transmitting/receiving surface and a second acoustic lens layer on the side facing the ultrasonic wave transmitting/receiving surface. The first acoustic lens layer and the second acoustic lens layer have different attenuation coefficients. The interface between the first acoustic lens layer and the second acoustic lens layer is parallel to the ultrasonic wave transmitting/receiving surface.

FIG. 4

**Description**

BACKGROUND

1. Technical Field

The present invention relates to an ultrasonic device, an ultrasonic module, and an ultrasonic measurement apparatus.

2. Related Art

There is a known ultrasonic probe of related art including a vibrator that transmits and receives an ultrasonic wave on the basis of the piezoelectric effect of a piezoelectric body (JP-B-7-121158, for example).

The ultrasonic probe described in JP-B-7-121158 includes a vibrator and an acoustic lens disposed on the vibrator. The acoustic lens is formed of two acoustic lens layers having different attenuation coefficients and layered on each other sequentially from the side facing the vibrator. The thickness dimension of each of the acoustic lens layers is so set that the amount of ultrasonic wave passing through the acoustic lens is uniform across the acoustic lens in the in-plane direction that intersects the thickness direction of the acoustic lens.

Specifically, out of the two acoustic lens layers, the thickness dimension of the acoustic lens layer having a smaller attenuation coefficient is increased when the acoustic lens is thick and is decreased when the acoustic lens is thin. The amount of ultrasonic wave passing through the acoustic lens is thus made uniform in the in-plane direction of the acoustic lens. The ultrasonic wave transmittance of the acoustic lens is increased, and ultrasonic wave transmission/reception efficiency of the ultrasonic probe is therefore improved, as compared with a monolayer acoustic lens, by use of the acoustic lens layer having a smaller attenuation coefficient.

In a case where an acoustic lens formed of a plurality of layers as described above is used, the ultrasonic wave transmitted from a vibrator is reflected off the interface between the acoustic lens layers in some cases. In this process, since the interface between the acoustic lens layers is curved in accordance with a convex surface (or concave surface) of the acoustic lens, the ultrasonic wave is reflected off the interface in a direction according to the curvature of the interface and then reaches the vibrator again. The elapsed period before the ultrasonic wave reflected off the interface (hereinafter also referred to as interface reflected wave) reaches the vibrator again varies depending on the position where the interface reflected wave is reflected.

That is, the vibrator, when it detects ultrasonic wave under measurement that is reflected in a living body, could undesirably detect the interface reflected wave as well as the ultrasonic wave under measurement. In this case, as a result of the measurement, a plurality of peaks corresponding to the interface reflected waves as well as a peak corresponding to the ultrasonic wave under measurement are detected, or what is called tailing occurs, resulting in a decrease in distance resolution.

As described above, in the configuration of the related art, an attempt to improve the ultrasonic wave transmission/reception efficiency is made, but the distance resolution can undesirably decrease due to the tailing described above.

SUMMARY

An advantage of some aspects of the invention is to provide an ultrasonic device, an ultrasonic module, and an ultrasonic measurement apparatus that allow improvement in the transmission/reception efficiency and the distance resolution in the form of the following aspects or application examples.

An ultrasonic device according to this application example includes an ultrasonic transceiver having a flat, ultrasonic wave transmitting/receiving surface and an acoustic lens provided on the ultrasonic wave transmitting/receiving surface. The acoustic lens has a first acoustic lens layer on a side facing away from the ultrasonic wave transmitting/receiving surface and a second acoustic lens layer on a side facing the ultrasonic wave transmitting/receiving surface. The first acoustic lens layer and the second acoustic lens layer have different attenuation coefficients. An interface between the first acoustic lens layer and the second acoustic lens layer is parallel to the ultrasonic wave transmitting/receiving surface.

In this application example, the phrase of "an acoustic lens is provided on the ultrasonic wave transmitting/receiving surface" means that the acoustic lens is disposed in a portion that overlaps at least with the ultrasonic wave transmitting/receiving surface when viewed along the direction of a normal to the ultrasonic wave transmitting/receiving surface. For example, the phrase includes a situation in which another member, such as an acoustic matching layer, is disposed between the ultrasonic wave transmitting/receiving surface and the acoustic lens.

In this application example, the acoustic lens includes the first acoustic lens layer and the second acoustic lens layer having attenuation coefficients different from each other, and the interface between the first acoustic lens layer and the second acoustic lens layer is parallel to the flat, ultrasonic wave transmitting/receiving surface. In this configuration, even when an interface reflected wave occurs at the interface, a situation in which the ultrasonic transceiver detects the interface reflected wave at different points of time can be avoided, unlike in a configuration in which the interface described above is curved, that is, occurrence of tailing can be avoided. The distance resolution can therefore be improved by performing ultrasonic wave measurement by using the ultrasonic device.

Further, setting one of the attenuation coefficients of the first and second acoustic lens layers to be smaller than the other allows an increase in ultrasonic wave transmittance of the acoustic lens and hence improvement in ultrasonic wave transmission/reception efficiency.

In the ultrasonic device according to the application example, it is preferable that the ultrasonic transceiver includes a vibration film and a piezoelectric element provided on the vibration film, and that the attenuation coefficient of the second acoustic lens layer is smaller than the attenuation coefficient of the first acoustic lens layer.

In the application example with this configuration, the ultrasonic transceiver includes a vibration film and a piezoelectric element, and the piezoelectric element is driven to cause the vibration film to vibrate and transmit an ultrasonic wave, and the piezoelectric element detects vibration of the vibration film caused to vibrate by an ultrasonic wave to receive the ultrasonic wave. The thus configured ultrasonic transceiver has a small acoustic impedance as compared, for example, with an ultrasonic transceiver so configured that a bulk-shaped piezoelectric body is caused to vibrate in place of the vibration film to transmit an ultrasonic wave and the vibration of the piezoelectric body excited by an ultrasonic wave is detected. In the application example with the configuration described above, in which out of the acoustic lens layers, the second acoustic lens layer disposed on the side facing the ultrasonic transceiver has an attenuation coefficient smaller than that of the first acoustic lens layer, the ultrasonic wave is allowed to efficiently propagate even in a case where an ultrasonic transceiver having a relatively small acoustic impedance is used.

In the ultrasonic device according to the application example, it is preferable that, in a portion that overlaps with the ultrasonic transceiver when viewed along a direction of a normal to the ultrasonic wave transmitting/receiving surface, a thickness dimension of the second acoustic lens layer along the direction of the normal is greater than a thickness dimension of the first acoustic lens layer along the direction of the normal.

In the application example with this configuration, in the portion that overlaps with the ultrasonic transceiver when viewed in the direction of the normal, that is, in the portion where the ultrasonic wave propagates, the thickness dimension of the second acoustic lens layer, which has an attenuation coefficient smaller than that of the first acoustic lens layer, is greater than the thickness dimension of the first acoustic lens layer. As a result, in the portion where the ultrasonic wave propagates, the ultrasonic wave transmittance can be further increased.

In the ultrasonic device according to the application example, it is preferable that $L=(\lambda/2)\times n$ is satisfied, where L represents a distance in a direction of a normal to the ultrasonic wave transmitting/receiving surface from the interface between the first acoustic lens layer and the second acoustic lens layer to the ultrasonic wave transmitting/receiving surface, $\lambda$ represents a wavelength of the ultrasonic wave transmitted from the ultrasonic transceiver, and n represents a positive integer.

The interface reflected wave that occurs at the interface between the first acoustic lens layer and the second acoustic lens layer is reflected off the ultrasonic wave transmitting/receiving surface and then passes through the interface in some cases. In such cases, out of reflected waves reflected in a living body, an ultrasonic wave resulting from the interface reflected wave is detected after a reflected wave that does not result from the interface reflected wave (that is, reflected wave under measurement), and a result of the measurement could contain tailing.

In contrast, in the application example having the configuration described above, occurrence of the tailing can be avoided, whereby the distance resolution can be improved. Specifically, since the acoustic impedance of the ultrasonic transceiver is small as described above, the phase of the interface reflected wave is reversed when reflected off the ultrasonic wave transmitting/receiving surface. Therefore, when the distance L between the interface and the ultrasonic wave transmitting/receiving surface satisfies the expression described above, the phase of the interface reflected wave reflected off the ultrasonic wave transmitting/receiving surface and then incident on the interface is opposite the phase of the ultrasonic wave transmitted from the ultrasonic transceiver and passing through the interface. The interface reflected wave can therefore be canceled, and occurrence of the tailing resulting from the interface reflected wave can be avoided, whereby the distance resolution can be improved.

In the ultrasonic device according to the application example, it is preferable that the first acoustic lens layer has a recessed section on a side facing the ultrasonic wave transmitting/receiving surface, and that the second acoustic lens layer is disposed in the recessed section.

In the application example with this configuration, the second acoustic lens layer is disposed in the recessed section of the first acoustic lens layer. In this configuration, the acoustic lens can be formed, for example, by forming the first acoustic lens layer and then forming the second acoustic lens layer in the recessed section. Therefore, forming a recessed section according to the position where the second acoustic lens layer is disposed and the shape of the second acoustic lens layer in the first acoustic lens layer allows the second acoustic lens layer to be readily formed. Further, the degree of intimate contact between the first acoustic lens layer and the second acoustic lens layer can be readily improved.

An ultrasonic module according to an application example of the invention includes an ultrasonic device including an ultrasonic transceiver having a flat, ultrasonic wave transmitting/receiving surface and an acoustic lens provided on the ultrasonic wave transmitting/receiving surface and a circuit substrate on which the ultrasonic device is provided. The acoustic lens has a first acoustic lens layer on a side facing away from the ultrasonic wave transmitting/receiving surface and a second acoustic lens layer on a side facing the ultrasonic wave transmitting/receiving surface. The first acoustic lens layer and the second acoustic lens layer have different attenuation coefficients. An interface between the first acoustic lens layer and the second acoustic lens layer is parallel to the ultrasonic wave transmitting/receiving surface.

In this application example, the acoustic lens includes the first acoustic lens layer and the second acoustic lens layer having attenuation coefficients different from each other, and the interface between the first acoustic lens layer and the second acoustic lens layer is parallel to the flat, ultrasonic wave transmitting/receiving surface.

In this configuration, since the interface described above is parallel to the flat, ultrasonic wave transmitting/receiving surface, as in the application example according to the ultrasonic device described above, occurrence of tailing resulting from the interface reflected wave that occurs at a curved interface can be avoided, unlike a configuration in which the interface is curved. The distance resolution achieved when ultrasonic wave measurement is performed by using the ultrasonic module can therefore be improved.

Further, setting one of the attenuation coefficients of the first and second acoustic lens layers to be smaller than the other allows an increase in ultrasonic wave transmittance of the acoustic lens and hence improvement in ultrasonic wave transmission/reception efficiency.

An ultrasonic measurement apparatus according to an application example of the invention includes an ultrasonic device including an ultrasonic transceiver having a flat, ultrasonic wave transmitting/receiving surface and an acoustic lens provided on the ultrasonic wave transmitting/receiving surface and a control section that controls the ultrasonic device. The acoustic lens has a first acoustic lens layer on a side facing away from the ultrasonic wave transmitting/receiving surface and a second acoustic lens layer on a side facing the ultrasonic wave transmitting/receiving surface. The first acoustic lens layer and the second acoustic lens layer have different attenuation coefficients. An interface between the first acoustic lens layer and the second acoustic lens layer is parallel to the ultrasonic wave transmitting/receiving surface.

In this application example, the acoustic lens includes the first acoustic lens layer and the second acoustic lens layer having attenuation coefficients different from each other, and the interface between the first acoustic lens layer and the second acoustic lens layer is parallel to the flat, ultrasonic wave transmitting/receiving surface.

In this configuration, since the interface described above is parallel to the flat, ultrasonic wave transmitting/receiving surface, as in the application example according to the ultrasonic device described above, occurrence of tailing resulting from the interface reflected wave that occurs at a curved interface can be avoided, unlike a configuration in which the interface is curved. The distance resolution achieved when ultrasonic wave measurement is performed by using the ultrasonic measurement apparatus can therefore be improved.

Further, setting one of the attenuation coefficients of the first and second acoustic lens layers to be smaller than the other allows an increase in ultrasonic wave transmittance of the acoustic lens and hence improvement in ultrasonic wave transmission/reception efficiency.

BRIEF DESCRIPTION OF THE DRAWINGS

The invention will be described with reference to the accompanying drawings, wherein like numbers reference like elements.

Fig. 1 is shows a schematic configuration of an ultrasonic measurement apparatus according to an embodiment.
Fig. 2 is a plan view showing a schematic configuration of an ultrasonic sensor in the embodiment.
Fig. 3 is a plan view of an element substrate of an ultrasonic device in the embodiment viewed from the side facing a sealing plate.
Fig. 4 is a cross-sectional view of the ultrasonic device taken along the line A-A in Fig. 3.
Fig. 5 is a cross-sectional view showing a schematic configuration of an ultrasonic device in Comparative Example.
Fig. 6A shows an example of a result of measurement performed by the ultrasonic device according to Comparative Example, and Fig. 6B shows an example of a result of measurement performed by the ultrasonic device according to the embodiment.
Fig. 7 is a cross-sectional view showing a schematic configuration of the ultrasonic device in the embodiment.

DESCRIPTION OF EXEMPLARY EMBODIMENTS

An ultrasonic apparatus according to an embodiment will be described below with reference to the drawings.

Configuration of ultrasonic measurement apparatus

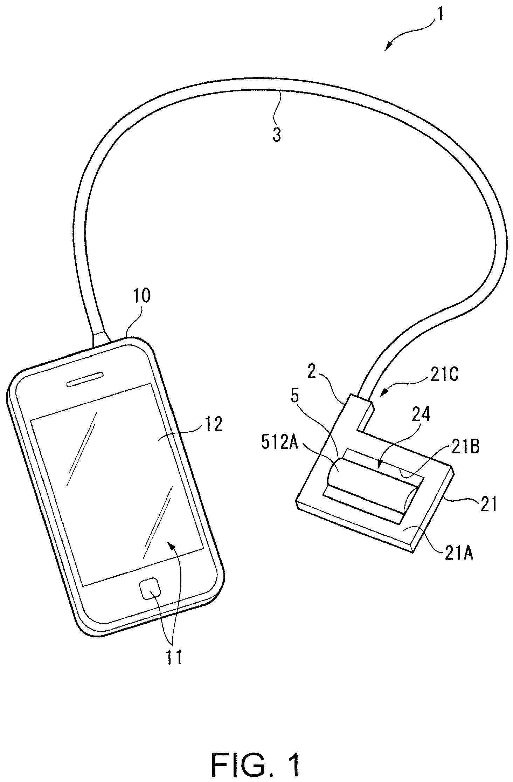

Fig. 1 is a perspective view showing a schematic configuration of an ultrasonic measurement apparatus 1 according to the present embodiment.
The ultrasonic measurement apparatus 1 according to the present embodiment corresponds to an electronic apparatus and includes an ultrasonic probe 2 and a controller 10, which is electrically connected to the ultrasonic probe 2 via a cable 3, as shown in Fig. 1.

The ultrasonic measurement apparatus 1 is so configured that the ultrasonic probe 2 is brought into contact with a surface of a living body (human body, for example) and the ultrasonic probe 2 transmits an ultrasonic wave into the living body. The ultrasonic probe 2 then receives the ultrasonic wave reflected off an organ in the living body, and the ultrasonic measurement apparatus 1 acquires an internal tomographic image in the living body, measures the state of the organ (blood flow therein, for example) in the living body, and performs other types of measurement on the basis of the received signal.

Configuration of controller

The controller 10 includes, for example, an operation section 11 and a display section 12, as shown in Fig. 1. The controller 10 further includes, although not shown, a storage section formed, for example, of a memory and a computation section formed, for example, of a CPU (central processing unit). The controller 10 causes the computation section to read and execute a variety of programs stored in the storage section to, for example, output an instruction for controlling drive operation of the ultrasonic probe 2, form an image of an internal structure in the living body on the basis of the received signal inputted from the ultrasonic probe 2 and cause the display section 12 to display the image, and measure information on the living body, such as blood flow, and cause the display section 12 to display the measured information. That is, the controller 10 corresponds to a control section. The controller 10 may, for example, be a tablet terminal, a smartphone, a personal computer, or any other terminal device or may instead be a dedicated terminal device for operating the ultrasonic probe 2.

Configuration of ultrasonic probe

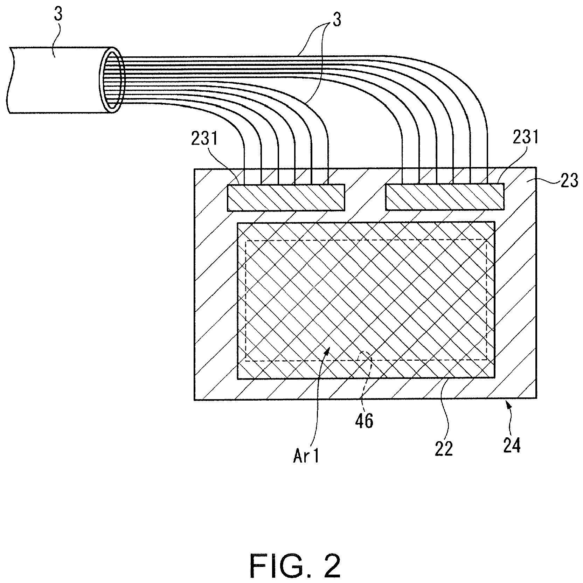

Fig. 2 is a plan view showing a schematic configuration of an ultrasonic sensor 24 in the ultrasonic probe 2.

The ultrasonic probe 2 includes an enclosure 21 (see Fig. 1), an ultrasonic device 22, which is provided in the enclosure 21, and a wiring substrate 23, on which a driver circuit and other components for controlling the ultrasonic device 22 are provided. The ultrasonic device 22 and the wiring substrate 23 form the ultrasonic sensor 24 (corresponding to ultrasonic module).

Configuration of enclosure

The enclosure 21 is formed in a box-like shape rectangular in a plan view and has a sensor window 21B provided in one surface perpendicular to the thickness direction (sensor surface 21A), and part of the ultrasonic device 22 is exposed through the sensor window 21B, as shown in Fig. 1. A passage hole 21C, through which the cable 3 passes, is provided in part of the enclosure 21 (side surface in the example shown in Fig. 1), and the cable 3 is inserted through the passage hole 21C into the enclosure 21 and connected to connectors 231 (see Fig. 2) on the wiring substrate 23. The gap between the cable 3 and the passage hole 21C is filled, for example, with a resin material for waterproofness.

In the present embodiment, the configuration in which the ultrasonic probe 2 is connected to the controller 10 with the cable 3 is shown by way of example, but the cable connection is not necessarily employed, and the ultrasonic probe 2 may, for example, be connected to the controller 10 in wireless communication, or a variety of configurations of the controller 10 may be provided in the ultrasonic probe 2.

Configuration of wiring substrate

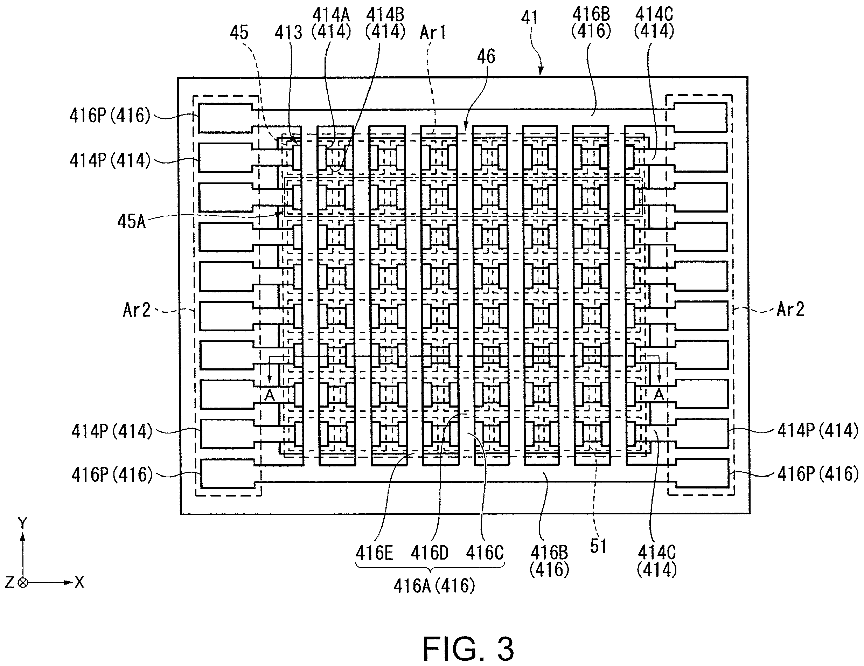

The wiring substrate 23 corresponds to a circuit substrate and includes a terminal section electrically connected to electrode pads 414P and 416P (see Fig. 3), with which the ultrasonic device 22 is provided.

The wiring substrate 23 is provided with the driver circuit and other components for driving the ultrasonic device 22. Specifically, the wiring substrate 23 is provided with a transmission circuit for transmitting an ultrasonic wave from the ultrasonic device 22, a reception circuit that processes a received signal when the ultrasonic device 22 receives an ultrasonic wave, and other circuits. The wiring substrate 23 is connected to the controller 10 via the cable 3 and other components and drives the ultrasonic device 22 on the basis of an instruction from the controller 10.

Configuration of ultrasonic device

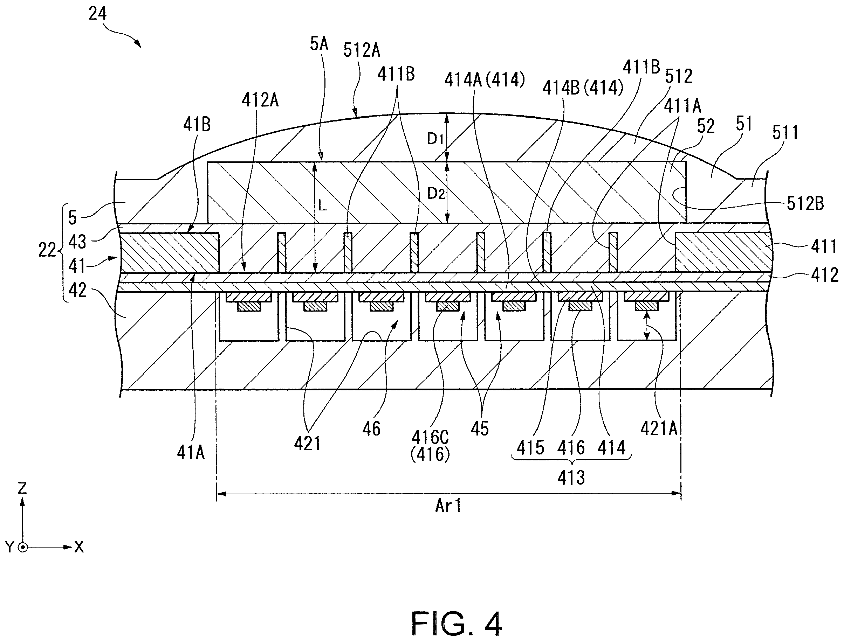

Fig. 3 is a plan view of an element substrate 41 in the ultrasonic device 22 viewed from the side facing a sealing plate 42. Fig. 4 is a cross-sectional view of the ultrasonic device 22 taken along the line A-A in Fig. 3.

The ultrasonic device 22 is formed of the element substrate 41, the sealing plate 42, an acoustic matching layer 43, and an acoustic lens 5, as shown in Fig. 4.

Configuration of element substrate

The element substrate 41 includes a substrate main body 411, a vibration film 412, which is provided on a side of the substrate main body 411 or the side facing the sealing plate 42, and piezoelectric elements 413, which are layered on the vibration film 412, as shown in Fig. 4. In preparation for the following description, a surface of the element substrate 41 or the surface facing the sealing plate 42 is referred to as a rear surface 41A. Further, a surface of the vibration film 412 or the surface facing away from the sealing plate 42 is referred to as an ultrasonic wave transmitting/receiving surface 412A. In a plan view of the element substrate 41 viewed along the substrate thickness direction, a central area of the element substrate 41 forms an array area Ar1, and a plurality of ultrasonic transducers 45 are arranged in a matrix in the array area Ar1.

The substrate main body 411 is a semiconductor substrate made, for example, of Si. Opening sections 411A, which correspond to the ultrasonic transducers 45, are provided in the array area Ar1 of the substrate main body 411. The opening sections 411A are closed by the vibration film 412, which is provided on the rear surface 41A of the substrate main body 411.

The vibration film 412 is, for example, made of $SiO_2$ or formed of a laminate made of $SiO_2$ and $ZrO_2$ and so provided as to cover the entire rear surface 41A of the substrate main body 411. The thickness dimension of the vibration film 412 is sufficiently smaller than the thickness dimension of the substrate main body 411. In the case where the substrate main body 411 is made of Si and the vibration film 412 is made of $SiO_2$, the vibration film 412 having a desire thickness dimension can be readily formed, for example, by oxidization of the rear surface 41A of the substrate main body 411. In this case, the opening sections 411A can be readily formed in the process of etching the substrate main body 411 with the vibration film 412 made of $SiO_2$ serving as an etching stopper.

The piezoelectric elements 413, each of which is a laminate of a lower electrode 414, a piezoelectric film 415, and an upper electrode 416, are provided on the vibration film 412, which closes the opening sections 411A, (on the side facing the rear surface 41A), as shown in Fig. 4. The vibration film 412, which closes the opening sections 411A, and the piezoelectric elements 413 form the individual ultrasonic transducers 45.

Each of the thus formed ultrasonic transducers 45, in which the vibration film 412 in the opening area of the opening section 411A is caused to vibrate by application of predetermined-frequency rectangular-waveform voltage to the segment between the lower electrode 414 and the upper electrode 416, can transmit an ultrasonic wave through the ultrasonic wave transmitting/receiving surface 412A. When the ultrasonic wave reflected off an object and incident through the ultrasonic wave transmitting/receiving surface 412A causes the vibration film 412 to vibrate, a potential difference is produced between the upper and lower surfaces of each of the piezoelectric films 415. Detection of the potential difference produced between the lower electrode 414 and the upper electrode 416 therefore allows detection of the received ultrasonic wave.

In the present embodiment, the plurality of ultrasonic transducers 45 described above are arranged in the predetermined array area Ar1 of the element substrate 41 along an X direction (slicing direction) and a Y direction (scanning direction) that intersects the X direction (perpendicular to the X direction in the present embodiment) to form an ultrasonic transducer array 46, as shown in Fig. 3. The ultrasonic transducer array 46 corresponds to an ultrasonic transceiver.

Each of the lower electrodes 414 is formed linearly along the X direction. That is, each of the lower electrodes 414 is so provided as to extend across a plurality of the ultrasonic transducers 45 arranged along the X direction and is formed of lower electrode main bodies 414A, which are located between the piezoelectric films 415 and the vibration film 412, lower electrode lines 414B, which link adjacent lower electrode main bodies 414A with each other, and lower terminal electrode lines 414C, which are drawn to terminal areas Ar2 outside the array area Ar1. Therefore, in the ultrasonic transducers 45 aligned along the X direction, the lower electrode 414 is kept at the same potential.

The lower terminal electrode lines 414C extend to the terminal areas Ar2 outside the array area Ar1 and form first electrode pads 414P in the terminal areas Ar2. The first electrode pads 414P are connected to the terminal sections provided on the wiring substrate.

On the other hand, the upper electrode 416 has element electrode sections 416A, each of which is so provided as to extend across a plurality of the ultrasonic transducers 45 aligned along the Y direction, and common electrode sections 416B, which link the ends of the plurality of element electrode sections 416A with one another, as shown in Fig. 3. Each of the element electrode sections 416A has upper electrode main bodies 416C, which are layered on the piezoelectric films 415, upper electrode lines 416D, which link adjacent upper electrode main bodies 416C with each other, and upper terminal electrodes 416E, which extend along the Y direction outward from the ultrasonic transducers 45 arranged at opposite ends in the Y direction.

The common electrode sections 416B are provided in a +Y-side end portion and a -Y-side end portion of the array area Ar1. The +Y-side common electrode section 416B connects the upper terminal electrodes 416E that extend toward the +Y side from the ultrasonic transducers 45 provided in the +Y-side end portion, out of the plurality of ultrasonic transducers 45 provided along the Y direction, to one another. The -Y-side common electrode section 416B connects the upper terminal electrodes 416E that extend toward the -Y side to one another. Therefore, in the ultrasonic transducers 45 in the array area Ar1, the upper electrode 416 is kept at the same potential. Further, the pair of common electrode sections 416B are provided along the X direction, and the ends of the common electrode sections 416B are drawn out of the array area Ar1 to the terminal areas Ar2. The common electrode sections 416B in the terminal areas Ar2 form second electrode pads 416P, which are connected to the terminal sections on the wiring substrate.

In the ultrasonic transducer array 46 described above, the ultrasonic transducers 45 aligned in the X direction and linked with one another by the corresponding lower electrode 414 form a single ultrasonic transducer group 45A, and the ultrasonic transducer group 45A is repeated multiple times along the Y direction to form a one-dimensional array structure.

Configuration of sealing plate

The sealing plate 42 is formed, for example, in the same planar shape as that of the element substrate 41 when viewed in the thickness direction and is formed of a semiconductor substrate made, for example, of silicon, or an insulator substrate. The material and the thickness of the sealing plate 42, which affect the frequency characteristic of the ultrasonic transducers 45, are preferably set on the basis of the center frequency of the ultrasonic wave transmitted and received by the ultrasonic transducers 45.

The sealing plate 42 has a plurality of recessed grooves 421 formed in an array counter area that faces the array area Ar1 of the element substrate 41, and the plurality of recessed grooves 421 correspond to the opening sections 411A of the element substrate 41. Therefore, an area of the vibration film 412 or the area caused to vibrate by the ultrasonic transducer 45 (opening section 411A) faces a gap 421A having a predetermined dimension provided between the corresponding recessed groove 421 and the element substrate 41, whereby the vibration of the vibration film 412 is not inhibited. Further, an inconvenience (crosstalk) that occurs in a situation in which a backward wave from one ultrasonic transducer 45 is incident on another adjacent ultrasonic transducer 45 can be avoided.

When the vibration film 412 vibrates, an ultrasonic wave is emitted not only toward the opening sections 411A (ultrasonic wave transmitting/receiving surface 412A) but also toward, as a backward wave, the sealing plate 42 (rear surface 41A). The backward wave is reflected off the sealing plate 42 and radiated toward the vibration film 412 via the gaps 421A again. In this process, when the reflected backward wave and the ultrasonic wave emitted through the vibration film 412 toward the ultrasonic wave transmitting/receiving surface 412A are out of phase with each other, the ultrasonic wave attenuates. To address the problem, in the present embodiment, the groove depth of each of the recessed grooves 421 is so set that the acoustic distance in the gap 421A is an odd multiple of one-fourth the wavelength $\lambda$ ($\lambda/4$) of the ultrasonic wave. In other words, the thickness dimensions of the element substrate 41 and the sealing plate 42 are set in consideration of the wavelength $\lambda$ of the ultrasonic wave emitted from the ultrasonic transducers 45.

The sealing substrate 42 may further, for example, so configured that opening sections (not shown) are provided in correspondence with the electrode pad 414P and 416P provided in the terminal areas Ar2 of the element substrate 41 and in the positions facing the terminal areas Ar2. In this case, providing the opening sections with through electrodes (TSV: through-silicon via) passing through the sealing substrate 42 in the thickness direction thereof allows the electrode pads 414P and 416P to be connected to the terminal sections on the wiring substrate via the through electrodes. Further, for example, a configuration in which FPCs (flexible printed circuits), cable lines, wires, or other lines are inserted into the opening sections to connect the electrode pads 414P and 416P to the wiring substrate may be employed.

Configuration of acoustic matching layer

The acoustic matching layer 43 is provided on the side facing the ultrasonic wave transmitting/receiving surface 412A, as shown in Fig. 4. Specifically, the acoustic matching layer 43 fills the opening sections 411A of the element substrate 41 and has a predetermined thickness dimension measured from the ultrasonic wave transmitting/receiving surface 412A. The acoustic matching layer 43 along with the acoustic lens 5, which will be described later, allows the ultrasonic wave transmitted from the ultrasonic transducers 45 to efficiently propagate through a living body, which is an object under measurement, and the ultrasonic wave reflected in the living body to efficiently propagate back to the ultrasonic transducers 45. To this end, the acoustic impedance of the acoustic matching layer 43 is set to be an intermediate value between the acoustic impedance of the ultrasonic transducers 45 in the element substrate 41 and the acoustic impedance of the living body. Examples of a material having the intermediate acoustic impedance described above may include silicone and other resin materials.

Configuration of acoustic lens

The acoustic lens 5 is provided on the acoustic matching layer 43 and includes a first acoustic lens layer 51 and a second acoustic lens layer 52, which is disposed on a side of the first acoustic lens 51 or the side facing the ultrasonic wave transmitting/receiving surface 412A (-Z side). The acoustic lens 5 is exposed to the outside through the sensor window 21B of the enclosure 21, as shown in Fig. 1. The acoustic lens 5, when the first acoustic lens layer 51 is caused to come into intimate contact with a surface of the living body, causes the ultrasonic wave transmitted from the ultrasonic transducers 45 to efficiently converge in the living body via the acoustic matching layer 43 and further causes the ultrasonic wave reflected in the living body to efficiently propagate back to the ultrasonic transducers 45.

The first acoustic lens layer 51 includes a flat plate section 511 and a protruding section 512, which protrudes from the flat plate section 511 toward the side opposite the ultrasonic wave transmitting/receiving surface 412A, as shown in Fig. 4.

The flat plate section 511 is a plate-shaped portion disposed in a region outside the array area Ar1 in a plan view viewed along the direction of a normal to the ultrasonic wave transmitting/receiving surface 412A and on the acoustic matching layer 43.

The protruding section 512 has a cylindrical surface 512A, which protrudes toward the side opposite the ultrasonic wave transmitting/receiving surface 412A (side facing living body), and a recessed section 512B, which opens toward the ultrasonic wave transmitting/receiving surface 412A, and the protruding section 512 protrudes through the sensor window 21B.

The cylindrical surface 512A is a surface having an arcuate shape in a cross-sectional view taken along the X direction (slicing direction) and having a linear shape in a cross-sectional view taken along the Y direction (scanning direction). The curvature of the cylindrical surface 512A is determined in accordance with the focal position of the ultrasonic wave transmitted from each of the ultrasonic transducer groups 45A. The dimension of the protruding section 512 in the X direction, that is, the X-direction dimension of an area where the cylindrical surface 512A is formed is greater than at least the array area Ar1. The ultrasonic wave transmitted from each of the ultrasonic transducer groups 45A disposed in the array area Ar1 can thus efficiently converge into the focal position.

The recessed section 512B is formed in a portion that covers the array area Ar1 in the plan view viewed along the direction of a normal to the ultrasonic wave transmitting/receiving surface 412A, and the dimension of the opening of the recessed section 512B is greater than the array area Ar1. The recessed section 512B has a flat bottom surface roughly parallel to the ultrasonic wave transmitting/receiving surface 412A. The bottom surface of the recessed section 512B is an interface 5A between the second acoustic lens layer 52, which is disposed in the recessed section 512B as will be described later, and the first acoustic lens layer 51.

The first acoustic lens layer 51 described above is made of a material having an intermediate acoustic impedance between those of the ultrasonic transducers 45 in the element substrate 41 and the living body. Further, the first acoustic lens layer 51 is preferably made of a material having Shore hardness greater than that of the second acoustic lens layer 52. The thus formed first acoustic lens layer 51 can suppress friction resulting from the contact with the living body.

The material of which the first acoustic lens layer 51 can, for example, be a millable-type silicone rubber. The millable-type silicone rubber is formed, for example, of a silicone rubber having a dimethylpolysiloxane structure containing a vinyl group to which silica and a vulcanizing agent are added. Specifically, silica is mixed with the silicone rubber in the form of silica particles having a weight average particle diameter ranging from 15 to 30 $\mu$m and having a silica/silicone rubber mass ratio greater than or equal to 40 mass% but smaller than or equal to 50 mass%. The vulcanizing agent can, for example, be 2,5-dimethyl-2,5-di-tertially butyl peroxyhexane.

The second acoustic lens layer 52 is disposed in the recessed section 512B of the first acoustic lens layer 51. That is, the second acoustic lens layer 52 is disposed in a portion that overlaps with the array area Ar1 in the plan view viewed along the direction of a normal to the ultrasonic wave transmitting/receiving surface 412A (direction parallel to Z direction) and has an outer dimension greater than that of the array area Ar1. As a result, the ultrasonic wave transmitted from the ultrasonic transducers 45 disposed in the array area Ar1 is allowed to propagate to the first acoustic lens layer 51 via the second acoustic lens layer 52.

A surface of the second acoustic lens layer 52 or the surface facing away from the ultrasonic wave transmitting/receiving surface 412A is the interface 5A between the first acoustic lens layer 51 and the second acoustic lens layer 52 and is roughly parallel to the ultrasonic wave transmitting/receiving surface 412A. A surface of the second acoustic lens layer 52 or the surface facing the ultrasonic wave transmitting/receiving surface 412A is a flat surface flush with a surface of the flat plate section 511 of the first acoustic lens layer 51 or the surface facing the array area Ar1.

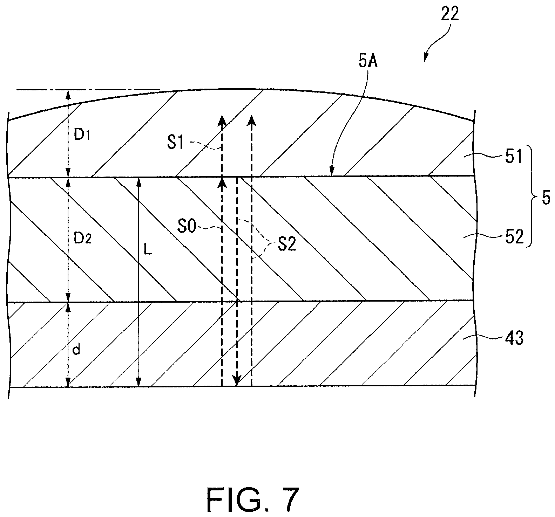

The thickness D2 of the second acoustic lens layer 52 is greater than the thickness D1 of the first acoustic lens layer 51. The thickness D1 of the first acoustic lens layer 51 is assumed to be the maximum thickness of the protruding section 512 (see Fig. 7). Attenuation of the ultrasonic wave can therefore be further suppressed, as compared with a configuration in which the thickness D1 of the first acoustic lens layer 51 is greater than the thickness D2 of the second acoustic lens layer 52.

The second acoustic lens layer 52 is made of a material having an attenuation coefficient smaller than that of the first acoustic lens layer 51 and also having an intermediate acoustic impedance between those of the ultrasonic transducers 45 and the living body. The material of which the second acoustic lens layer 52 is made can, for example, be an RTV silicone rubber containing no filler, such as silica. As a result, the attenuation coefficient of the second acoustic lens layer 52 disposed in a position where the ultrasonic wave propagates in the acoustic lens 5 can be smaller than the attenuation coefficient of the first acoustic lens layer 51, whereby attenuation of the ultrasonic wave can be suppressed.

In the acoustic lens 5 configured as described above, the distance L between the ultrasonic wave transmitting/receiving surface 412A and the interface 5A satisfies the following Expression (1), where $\lambda$ represents the wavelength of the ultrasonic wave transmitted from the ultrasonic transducers 45 and n represents an integer greater than or equal to 1. The distance L is the sum of the thickness d of the acoustic matching layer 43 (see Fig. 7) and the thickness D2 of the second acoustic lens layer. That is, in the present embodiment, the thickness d of the acoustic matching layer 43 and the thickness D2 of the second acoustic lens layer are so set that the distance L satisfies the following Expression (1). An advantageous effect provided when the distance L satisfies the following Expression (1) will be described later.
Numerical expression 1

$$L = (\lambda / 2) \times n \quad (1)$$

The acoustic lens 5 described above can be formed, for example, in compression molding using a die made, for example, of a metal. For example, a first die that forms the outer shape of the first acoustic lens layer 51 is first filled with a fluid material of which the first acoustic lens layer 51 is made, and the material is then allowed to cure. The interior of the recessed section 512B of the thus formed first acoustic lens layer 51 is filled with a fluid material of which the second acoustic lens layer 52 is made. A die for making a surface of the second acoustic lens layer 52 or the surface facing the array area flat is disposed in a portion that covers the recessed section 512B, and the material of which the second acoustic lens layer 52 is allowed to cure. After the recessed section 512B is formed in the first acoustic lens layer 51 as described above, the second acoustic lens layer 52 is formed in the recessed section 512B, whereby the degree of intimate contact between the first acoustic lens layer 51 and the second acoustic lens layer 52 can be improved.

Tailing suppression achieved by ultrasonic device 22

In the ultrasonic device 22 in the present embodiment, in which the interface 5A is roughly parallel to the ultrasonic wave transmitting/receiving surface 412A, occurrence of tailing can be suppressed (first effect), as will be described later.

Further, in the ultrasonic device 22, the occurrence of tailing can also be suppressed when the distance L between the interface 5A and the ultrasonic wave transmitting/receiving surface 412A satisfies Expression (1) described above (second effect).

The first and second effects described above provided by the ultrasonic device 22 will be described below.

First effect

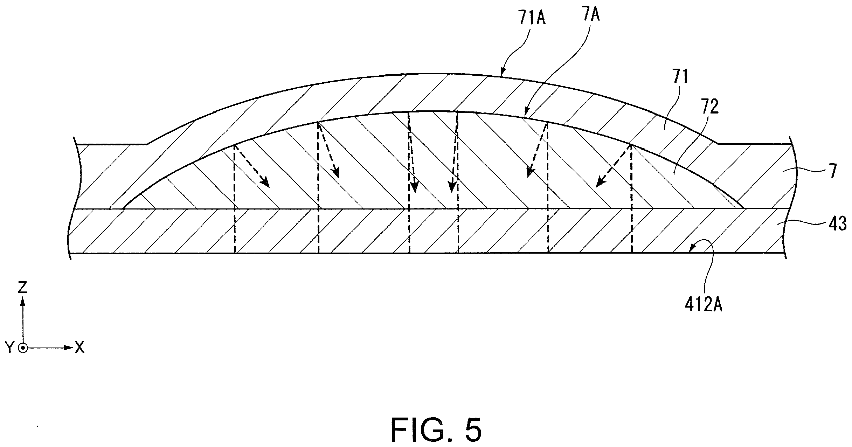

Fig. 5 shows a schematic configuration of a cross section of an acoustic lens 7 in Comparative Example.

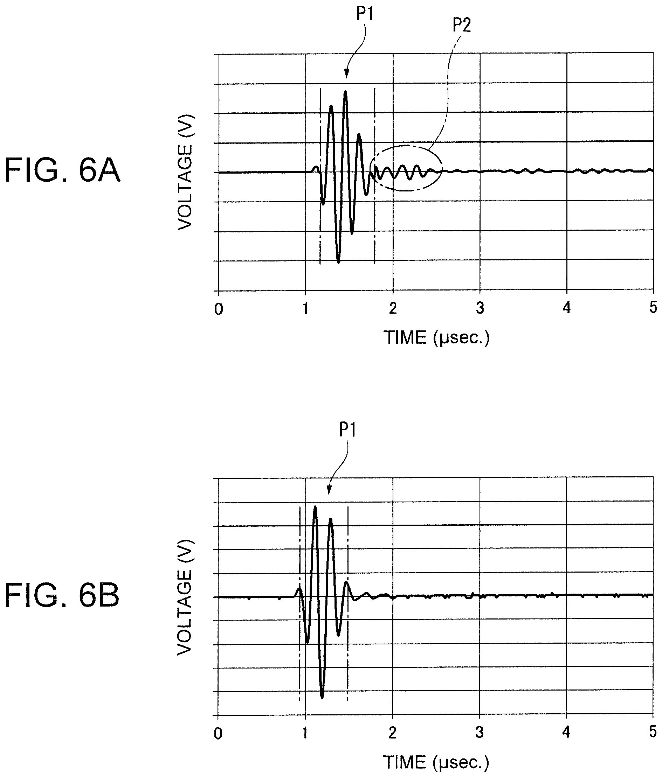

Figs. 6A and 6B show exemplary measurement results of ultrasonic wave measurement performed by the ultrasonic device. Fig. 6A shows a result of the measurement performed by the ultrasonic device according to Comparative Example described above, and Fig. 6B shows a result of the measurement performed by the ultrasonic device 22 according to the present embodiment.

The acoustic lens 7 shown in Fig. 5 includes a first acoustic lens layer 71 and a second acoustic lens layer 72 and differs from the acoustic lens 5 in the present embodiment in that an interface 7A between the first acoustic lens layer 71 and the second acoustic lens layer 72 is curved. Fig. 5 shows, by way of example, the acoustic lens 7 in which the interface 7A is curved along a curved surface 71A of the first acoustic lens layer 71. The first effect will be described below by comparing the ultrasonic device with the ultrasonic device including the acoustic lens 7.

In the ultrasonic device including the acoustic lens 7 according to Comparative Example shown in Fig. 5, the ultrasonic wave transmitted in the Z direction from each of the ultrasonic transducers 45 in the ultrasonic transducer groups 45A propagates through the second acoustic lens layer 72 of the acoustic lens 7 and is then incident on the interface 7A between the first acoustic lens layer 71 and the second acoustic lens layer 72. Part of the ultrasonic wave incident on the interface 7A is reflected off the interface 7A in some cases, as shown in Fig. 5. When the ultrasonic wave reflected off the interface 7A (hereinafter also referred to as interface reflected wave) is received with the ultrasonic transducers 45, a plurality of peaks P2, which differ from a peak P1 resulting from the reflected wave produced in the living body, are detected, as shown in Fig. 6A. What is called tailing thus occurs.

That is, since the interface 7A of the acoustic lens 7 is curved or is not parallel to the ultrasonic wave transmitting/receiving surface 412A, the interface reflected wave propagates in a direction that intersects the Z direction, as shown in Fig. 5. When the position where the ultrasonic wave is incident on the interface 7A varies along the X direction, the direction in which the interface reflected wave propagates (direction in which ultrasonic wave is reflected) varies. Further, since the distance L between the ultrasonic wave transmitting/receiving surface 412A and the interface 7A varies along the X direction, the propagation distance over which the ultrasonic wave travels after it is transmitted from the ultrasonic transducers 45 and reflected as the interface reflected wave and before it reaches the ultrasonic transducers 45 again varies depending on the position where the ultrasonic wave is incident on the interface 7A. The period required for the interface reflected wave to reach the ultrasonic transducers 45 therefore varies depending on the X-direction position where the ultrasonic wave is incident on the interface 7A.

When the interface reflected wave is received with the ultrasonic transducers 45, the plurality of peaks P2 are detected, that is, tailing occurs, as shown in Fig. 6A. When the tailing occurs, the accuracy of detection of the reflection position where a reflected wave is produced in the living body (distance resolution) decreases.

In contrast, in the ultrasonic device 22 including the acoustic lens 5 according to the present embodiment, the interface 5A of the acoustic lens 5 is roughly parallel to the ultrasonic wave transmitting/receiving surface 412A. Therefore, even when the interface reflected wave occurs at the interface 5A, the direction in which the interface reflected wave propagates is roughly parallel to the Z direction. Further, the distance between the ultrasonic wave transmitting/receiving surface 412A and the interface 5A is roughly fixed. The situation in which a plurality of peaks P2 resulting from interface reflected waves that reach the ultrasonic transducers 45 at different points of time are detected can be avoided, as shown in Fig. 6B. That is, occurrence of the tailing can be suppressed. The distance resolution of the ultrasonic device 22 can therefore be improved.

Second effect

Fig. 7 describes how the ultrasonic device 22 according to the present embodiment suppresses the tailing, and Fig. 7 diagrammatically shows a cross section of the ultrasonic device 22. Fig. 7 shows a simplified version of the configuration of the ultrasonic device 22.

In the ultrasonic device 22 according to the present embodiment, when the distance L from the ultrasonic wave transmitting/receiving surface 412A to the interface 5A satisfies Expression (1) described above, tailing that occurs when the interface reflected wave is reflected off the ultrasonic wave transmitting/receiving surface 412A, then reflected in the living body, and received with the ultrasonic transducers 45 can be suppressed.

That is, an ultrasonic wave S0 transmitted from the ultrasonic transducers 45 in the direction of a normal thereto and incident on the interface 5A not only passes through the interface 5A to form an ultrasonic wave (first wave) S1 but also is reflected off the interface 5A to form an interface reflected wave (second wave) S2 in some cases. Out of the first and second waves, the second wave S2 is reflected off the ultrasonic wave transmitting/receiving surface 412A, then propagates through the acoustic lens 5 again, and exits into the living body in some cases. In ultrasonic wave measurement, a reflected wave or the first wave S1 reflected in the living body is measured. In the case described above, however, the tailing occurs in some cases when the second wave S2 is reflected in the living body and detected after the first wave S1 is detected.

However, in the present embodiment, in which the distance L from the ultrasonic wave transmitting/receiving surface 412A to the interface 5A satisfies Expression (1) described above, occurrence of the tailing resulting from the second wave S2 can be suppressed.

Specifically, the phase of the second wave S2 is reversed when it is reflected off the ultrasonic wave transmitting/receiving surface 412A. When the distance L satisfies Expression (1) described above, as described above, the phase of the second wave S2 is opposite the phase of the first wave S1 when the second wave S2 is incident on the interface 5A again. As a result, at least part of the second wave S2 can be canceled at the interface 5A. Occurrence of the tailing resulting from the second wave S2 can therefore be avoided, whereby the distance resolution of the ultrasonic device 22 can be improved.

Advantageous effects of present embodiment

The acoustic lens 5 includes the first acoustic lens layer 51 and the second acoustic lens layer 52 having attenuation coefficient different from each other, and the interface 5A between the first acoustic lens layer 51 and the second acoustic lens layer 52 is roughly parallel to the flat, ultrasonic wave transmitting/receiving surface 412A. The configuration can suppress, even when the interface reflected wave occurs at the interface 5A, the tailing that occurs when the interface reflected wave produced at a curved interface, for example, in the configuration in which the interface is curved (see Fig. 5), is detected with the ultrasonic transducer array 46 at different points of time, as described above. The distance resolution can therefore be improved by performing ultrasonic wave measurement by using the ultrasonic device 22.

Further, providing the second acoustic lens layer 52, which has an attenuation coefficient smaller than that of the first acoustic lens layer 51, allows an increase in ultrasonic wave transmittance of the acoustic lens 5 and hence improvement in ultrasonic wave transmission/reception efficiency.

The ultrasonic device 22 according to the present embodiment therefore allows simultaneous improvement in the ultrasonic wave transmission/reception efficiency and the distance resolution.

Further, the thickness dimension D2 of the second acoustic lens layer 52 is greater than the thickness dimension D1 of the first acoustic lens layer 51, whereby the ultrasonic wave transmittance can be further increased.

In the present embodiment, the second acoustic lens layer 52 has an outer dimension greater than that of the array area Ar1 in the plan view viewed along the direction of a normal to the ultrasonic wave transmitting/receiving surface 412A. The ultrasonic wave transmitted from the ultrasonic transducer array 46 is therefore allowed to efficiently propagate toward a living body.

The ultrasonic transducer array 46 includes the plurality of ultrasonic transducers 45, which includes the vibration film 412 and the piezoelectric elements 413 formed on the vibration film 412. Each of the ultrasonic transducers 45 has acoustic impedance smaller than, for example, the acoustic impedance of an ultrasonic transducer that includes no vibration film 412 but causes a bulk-shaped piezoelectric body to vibrate to transmit an ultrasonic wave and detects vibration of the piezoelectric body excited by an ultrasonic wave and the acoustic impedance a living body. In the present embodiment, out of the acoustic lens layers, the attenuation coefficient of the second acoustic lens layer 52, which is disposed on the side facing the ultrasonic transducer array 46, is set to be smaller than the attenuation coefficient of the first acoustic lens layer 51, whereby the ultrasonic wave is allowed to efficiently propagate even when an ultrasonic transducer array 46 having small acoustic impedance is used.

Further, in the present embodiment, the ultrasonic device 22 is so configured that the distance L from the interface 5A between the first acoustic lens layer 51 and the second acoustic lens layer 52 to the ultrasonic wave transmitting/receiving surface 412A satisfies Expression (1) described above. In the configuration, even when interface reflected wave occurs at the interface 5A as described above, at least part of the interface reflected wave can be canceled after the interface reflected wave is reflected off the ultrasonic wave transmitting/receiving surface 412A and when the interface reflected wave is incident on the interface 5A again. Occurrence of the interface reflected wave that is incident on the interface 5A again and then exits into a living body can therefore be avoided, whereby occurrence of tailing resulting from the interface reflected wave can be avoided.

The second acoustic lens layer 52 is disposed in the recessed section 512B of the first acoustic lens layer 51. In this configuration, the acoustic lens 5 can be formed, for example, by forming the first acoustic lens layer 51 and then forming the second acoustic lens layer 52 in the recessed section 512B. The second acoustic lens layer 52 can therefore be readily formed by forming, in the first acoustic lens layer 51, the recessed section 512B according to the position where the second acoustic lens layer 52 is disposed and the shape of the second acoustic lens layer 52. Further, the degree of intimate contact between the first acoustic lens layer 51 and the second acoustic lens layer 52 can be readily improved.

Variations

The embodiments described above are not limited to the configurations described in the embodiments, and changes, improvements, appropriate combination of the embodiments, and other modifications may be made.

For example, the above embodiment has been described with reference to the configuration in which the thickness dimension D2 of the second acoustic lens layer 52 is greater than the thickness dimension D1 of the first acoustic lens layer 51, but not necessarily in the invention. That is, the thickness dimension D1 of the first acoustic lens layer 51 may be greater than the thickness dimension D2 of the second acoustic lens layer 52 or may be equal to the thickness dimension D2 of the second acoustic lens layer 52. Also in these cases, disposing the second acoustic lens layer 52 having an attenuation coefficient smaller than that of the first acoustic lens layer 51 allows improvement in the ultrasonic wave transmittance.

The above embodiment has been described with reference to the configuration in which the second acoustic lens layer 52 is disposed in the recessed section 512B of the first acoustic lens layer 51, but not necessarily in the invention. For example, the first acoustic lens layer 51 may have no recessed section 512B but has a flat surface on the side facing the ultrasonic wave transmitting/receiving surface 412A, and the second acoustic lens 52 may be disposed along the flat surface of the first acoustic lens layer 51 on the side facing the ultrasonic wave transmitting/receiving surface 412A.

Further, in the embodiment described above, after the acoustic lens 5 is formed, the acoustic lens 5 is disposed on the acoustic matching layer 43. Instead, the second acoustic lens layer 52 may be integrated with the acoustic matching layer 43. That is, a material of which the acoustic matching layer 43 and the second acoustic lens layer 52 are made may be disposed on the ultrasonic wave transmitting/receiving surface 412A in the ultrasonic device 22, and the first acoustic lens layer 51 may then be disposed on the second acoustic lens layer forming material. In this case, for example, disposing or forming a member for positioning the first acoustic lens layer 51 on the +Z-side surface of an outer circumferential portion or any other portion of the element substrate 41 allows the thickness dimension of the second acoustic lens layer 52 and the posture (parallelism) of the first acoustic lens layer 51 with respect to the ultrasonic wave transmitting/receiving surface 412A to be appropriately set.

In the embodiment described above, the acoustic lens 5 includes the first acoustic lens layer 51 and the second acoustic lens layer 52, but not necessarily in the invention, and the acoustic lens 5 may have a configuration in which three or more acoustic lens layers are provided. Also in the case where three or more acoustic lens layers are provided, configuring the interfaces between the lens layers to be flat and parallel to the ultrasonic wave transmitting/receiving surface 412A can prevent occurrence of the tailing, as described above.

The above embodiment has been described with reference to the configuration in which the opening sections 411A are provided in the element substrate 41 and on the side facing an operation surface 41B, the piezoelectric elements 413 are provided in the element substrate 41 and on the side facing the rear surface 41A, and an ultrasonic wave is transmitted toward the operation surface 41B (opening sections 411A), as shown in Fig. 4, but not necessarily.

For example, the opening sections 411A may be provided in the element substrate 41 and on the side facing the rear surface 41A, the piezoelectric elements 413 may be provided in the element substrate 41 and on the side facing the operation surface 41B, and an ultrasonic wave may be transmitted toward the operation surface 41B (piezoelectric elements 413). Still instead, the opening sections 411A may be provided in the element substrate 41 on the side facing the operation surface 41B, and the piezoelectric elements 413 may be provided on groove bottoms surfaces (vibration film 412) of the opening sections 411A on the side facing the operation surface 41B. Still instead, the opening sections 411A may be provided in the element substrate 41 and on the side facing the rear surface 41A, and the piezoelectric elements 413 may be provided on groove bottom surfaces (vibration film 412) of the opening sections 411A on the side facing the rear surface 41A.

Further, the piezoelectric elements 413 are formed of a laminate of the lower electrodes 414, the piezoelectric films 415, and the upper electrode 416 laminated on each other in the thickness direction, by way of example, but not necessarily. For example, a pair of electrodes facing each other may be disposed on one side of each of the piezoelectric films 415 perpendicular to the thickness direction thereof. The electrodes may instead be so disposed along the side surface of the piezoelectric film extending along the thickness direction thereof as to sandwich the piezoelectric film.

The above embodiment has been described with reference to the configuration in which the ultrasonic transducers 45 include the vibration film 412 and the piezoelectric elements 413 formed of a laminate of the lower electrodes 414, the piezoelectric films 415, and the upper electrode 416 and disposed on the vibration film 412, but not necessarily in the invention. That is, a piezoelectric element having a bulk-shaped piezoelectric body may be used as each of the ultrasonic transducers, the bulk-shaped piezoelectric body may be caused to vibrate in place of the vibration film to transmit an ultrasonic wave, and vibration of the piezoelectric body excited by an ultrasonic wave may be detected. In this case, the ultrasonic wave transmitting/receiving surface is the living-body-side surface of the piezoelectric body.

Further, the thus configured ultrasonic transducer typically has acoustic impedance greater than that of a living body. Therefore, the acoustic impedance of each of a plurality of acoustic lens layers that form an acoustic lens is reduced with distance from the ultrasonic transducer toward a living body for efficient transmission and reception of an ultrasonic wave.

In the embodiment described above, an ultrasonic measurement apparatus directed to living body measurement is presented by way of example, but not necessarily in the invention. For example, the invention is applicable to an electronic apparatus that is directed to measurement of a variety of structures, detects defects of the structures, and inspects deterioration due to aging. Further, for example, the invention is applicable to an electronic apparatus that is directed to measurement of a semiconductor package, a wafer, and other objects and detects a defect of an object under measurement.

In addition, the specific structure in actual implementation of the invention may be an appropriate combination of the embodiments and the variations described above or may be changed as appropriate to any other structure to the extent that the advantage of the invention is achieved.

**Claims**

1. An ultrasonic device (22) comprising:

   an ultrasonic transceiver (46) having a flat, ultrasonic wave transmitting/receiving surface (412A); and
   an acoustic lens (5) provided on the ultrasonic wave transmitting/receiving surface (412A),
   wherein the acoustic lens (5) has a first acoustic lens layer (51) on a side facing away from the ultrasonic wave transmitting/receiving surface (412A) and a second acoustic lens layer (52) on a side facing the ultrasonic wave transmitting/receiving surface (412A),
   the first acoustic lens layer (51) and the second acoustic lens layer (52) have different attenuation coefficients, and
   an interface between the first acoustic lens layer (51) and the second acoustic lens layer (52) is parallel to the ultrasonic wave transmitting/receiving surface (412A).

2. The ultrasonic device (22) according to claim 1, wherein the ultrasonic transceiver (46) includes a vibration film (412) and a piezoelectric element (413) provided on the vibration film (412), and the attenuation coefficient of the second acoustic lens layer (52) is smaller than the attenuation coefficient of the first acoustic lens layer (51).

3. The ultrasonic device (22) according to claim 2, wherein in a portion that overlaps with the ultrasonic transceiver (46) when viewed along a direction of a normal to the ultrasonic wave transmitting/receiving surface (412A), a thickness dimension of the second acoustic lens layer (52) along the direction of the normal is greater than a thickness dimension of the first acoustic lens layer (51) along the direction of the normal.

4. The ultrasonic device (22) according to claim 2 or 3, wherein $L=(\lambda/2)\times n$ is satisfied, where L represents a distance in a direction of a normal to the ultrasonic wave transmitting/receiving surface (412A) from the interface between the first acoustic lens layer (51) and the second acoustic lens layer (52) to the ultrasonic wave transmitting/receiving surface (412A), $\lambda$ represents a wavelength of the ultrasonic wave transmitted from the ultrasonic transceiver (46), and n represents a positive integer.

5. The ultrasonic device (22) according to any one of claims 1 to 4, wherein the first acoustic lens layer (51) has a recessed section (512B) on a side facing the ultrasonic wave transmitting/receiving surface (412A), and the second acoustic lens layer (52) is disposed in the recessed section (512B).

6. An ultrasonic module (24) comprising:

an ultrasonic device (22) according to any one of claims 1 through 5; and
a circuit substrate (23) on which the ultrasonic device (22) is provided.

7. An ultrasonic measurement apparatus (1) comprising:

an ultrasonic device (22) according to any one of claims 1 through 5; and
a control section (10) that controls the ultrasonic device (22).

1

3

10

12

11

2

5

512A

21C

24

21B

21

21A

FIG. 1

FIG. 2

FIG. 3

FIG. 4

FIG. 5

FIG. 6A

FIG. 6B

FIG. 7

**EP 3 217 391 A1**

Europäisches
Patentamt
European
Patent Office
Office européen
des brevets

# EUROPEAN SEARCH REPORT

Application Number

EP 17 15 9574

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/031128 A1 (KIYOSE KANECHIKA [JP]) 4 February 2016 (2016-02-04) | 1,2,4-7 | INV. G10K11/30 |
| Y | * figures 12, 4, 1, 2 * <br> * paragraph [0063] - paragraph [0064] * <br> * paragraph [0020] * <br> * paragraph [0058] * | 3 | |
| X,P | EP 3 037 180 A1 (SAMSUNG MEDISON CO LTD [KR]) 29 June 2016 (2016-06-29) <br> * figures 1,4 * <br> * paragraph [0100] - paragraph [0102] * <br> * paragraph [0001] * | 1,2,5,7 | |
| X | EP 1 591 996 A2 (NIHON DEMPA KOGYO CO [JP]) 2 November 2005 (2005-11-02) <br> * figure 1 * <br> * paragraph [0019] - paragraph [0022] * | 1,2 | |
| Y | US 2015/257734 A1 (CHAGGARES NICHOLAS CHRISTOPHER [CA]) 17 September 2015 (2015-09-17) <br> * paragraph [0019] - paragraph [0020] * <br> * figures 3, 4 * | 3 | TECHNICAL FIELDS SEARCHED (IPC) G10K B06B A61B |
| A | US 2012/007472 A1 (TAI ALAN C [US] ET AL) 12 January 2012 (2012-01-12) <br> * paragraph [0007] * <br> * figure 1 * | 3 | |
| A | US 2014/265728 A1 (LI WEI [US] ET AL) 18 September 2014 (2014-09-18) <br> * figures 2A, 2B * <br> * paragraph [0021] * | 4 | |

-/--

The present search report has been drawn up for all claims

| Place of search | Date of completion of the search | Examiner |
|---|---|---|
| The Hague | 1 June 2017 | Pitzer, Hanna |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

21

Europäisches
Patentamt

European
Patent Office

Office européen
des brevets

**EUROPEAN SEARCH REPORT**

Application Number

EP 17 15 9574

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2013/046080 A1 (KONINKL PHILIPS ELECTRONICS NV [NL]; KUNKEL HARRY AMON [US]; CRUIKSHAN) 4 April 2013 (2013-04-04) * page 1, line 20-27; page 5 line 25 - page 6, line 4; page 8, line 20 - page 9, line 8; figure 1 * | 1,3 | |

-----

TECHNICAL FIELDS
SEARCHED      (IPC)

The present search report has been drawn up for all claims

| Place of search | Date of completion of the search | Examiner |
|---|---|---|
| The Hague | 1 June 2017 | Pitzer, Hanna |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 17 15 9574

This annex lists the patent family members relating to the patent documents cited in the above-mentioned European search report.
The members are as contained in the European Patent Office EDP file on
The European Patent Office is in no way liable for these particulars which are merely given for the purpose of information.

01-06-2017

| Patent document cited in search report | | Publication date | Patent family member(s) | | Publication date |
|---|---|---|---|---|---|
| US 2016031128 | A1 | 04-02-2016 | CN | 105310716 A | 10-02-2016 |
| | | | JP | 2016032572 A | 10-03-2016 |
| | | | US | 2016031128 A1 | 04-02-2016 |
| EP 3037180 | A1 | 29-06-2016 | CN | 105726059 A | 06-07-2016 |
| | | | EP | 3037180 A1 | 29-06-2016 |
| | | | KR | 20160079260 A | 06-07-2016 |
| | | | US | 2016187298 A1 | 30-06-2016 |
| EP 1591996 | A2 | 02-11-2005 | EP | 1591996 A2 | 02-11-2005 |
| | | | JP | 4523328 B2 | 11-08-2010 |
| | | | JP | 2005312583 A | 10-11-2005 |
| | | | US | 2005245829 A1 | 03-11-2005 |
| US 2015257734 | A1 | 17-09-2015 | CA | 2942379 A1 | 17-09-2015 |
| | | | CN | 106456111 A | 22-02-2017 |
| | | | EP | 3116405 A1 | 18-01-2017 |
| | | | JP | 2017507736 A | 23-03-2017 |
| | | | TW | 201539428 A | 16-10-2015 |
| | | | US | 2015257734 A1 | 17-09-2015 |
| | | | WO | 2015138796 A1 | 17-09-2015 |
| US 2012007472 | A1 | 12-01-2012 | NONE | | |
| US 2014265728 | A1 | 18-09-2014 | CN | 104226577 A | 24-12-2014 |
| | | | JP | 2014183589 A | 29-09-2014 |
| | | | US | 2014265728 A1 | 18-09-2014 |
| | | | US | 2017133004 A1 | 11-05-2017 |
| WO 2013046080 | A1 | 04-04-2013 | BR | 112014006785 A2 | 04-04-2017 |
| | | | CN | 103827960 A | 28-05-2014 |
| | | | EP | 2748813 A1 | 02-07-2014 |
| | | | JP | 5981998 B2 | 31-08-2016 |
| | | | JP | 2014531255 A | 27-11-2014 |
| | | | MX | 343899 B | 28-11-2016 |
| | | | RU | 2014117011 A | 10-11-2015 |
| | | | US | 2014204717 A1 | 24-07-2014 |
| | | | US | 2016098984 A1 | 07-04-2016 |
| | | | WO | 2013046080 A1 | 04-04-2013 |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 7121158 B